# EUROPÄISCHE PATENTANMELDUNG

(11) **EP 3 960 211 A1**
(43) Veröffentlichungstag der Anmeldung: **02.03.2022**
(21) Anmeldenummer: 20193479.1
(22) Anmeldetag: 28.08.2020
(51) Int. Cl.: A61L 9/14, A61L 9/20

(54) **VORRICHTUNG ZUM ENTKEIMEN VON FLUID, INSBESONDERE LUFT**

(71) Anmelder: Air Cleaner AG, 8544 Attikon (CH)
(72) Erfinder: Calzaferri, Pascal, 8266 Steckborn (CH)
(74) Vertreter: Keller Schneider Patent- und Markenanwälte AG

(57) **Zusammenfassung**

Die Erfindung betrifft eine Vorrichtung (100) zum Entkeimen von Fluid, mit einem Gehäuse (110), einem Fluideinlass (120) zum Einströmen von zu entkeimendem Fluid in das Gehäuse (110), einem Fluidauslass (130) zum Ausströmen von entkeimten Fluid aus dem Gehäuse (110), einer Brennkammer (200) mit einer UV-Lampeneinheit (210) zum Entkeimen von durchströmenden Fluid, wobei die Brennkammer (200) innenseitig zumindest teilweise verspiegelt ausgebildet ist, und wobei zwischen der Brennkammer (200) und einer ersten Gehäusewand (122) ein Fluideinlasskanal (124) sowie zwischen der Brennkammer (200) und einer zweiten Gehäusewand (132) ein Fluidauslasskanal (134) angeordnet ist.

## Beschreibung

### Technisches Gebiet

Die Erfindung betrifft eine Vorrichtung zum Entkeimen von Fluid, mit einem Gehäuse, einem Fluideinlass zum Einströmen von zu entkeimendem Fluid in das Gehäuse, einem Fluidauslass zum Ausströmen von entkeimten Fluid aus dem Gehäuse, und einer Brennkammer mit einer UV-Lampeneinheit zum Entkeimen von durchströmenden Fluid.

### Stand der Technik

Oberflächen in öffentlichen Räumen sind massgeblich an der Verbreitung von Krankheiten durch Bakterien und Viren beteiligt. In Zeiten eines erhöhten Ansteckungsrisikos ist eine intensive Reinigung unabdingbar. Eine besondere Herausforderung stellt dabei die Übertragung von Viren über Aerosole in der Raumluft dar. Im Stand der Technik bekannt sind Entkeimungsvorrichtung, die mittels UV-Strahlung Fluide wie Luft oder Wasser reinigen.

Nachteilig im Stand der Technik ist häufig die mangelnde Effizienz der bekannten Entkeimungsvorrichtungen. Die mangelnde Effizienz wirkt sich beispielsweise dadurch aus, dass Fluide wie Luft oder Wasser trotz Reinigung nicht vollständig keimfrei sind und ein Restrisiko für die Verbreitung von Krankheiten durch Restbestände der Bakterien oder Viren bestehen bleibt. Dies führt regelmässig dazu, dass zusätzlich kostenintensive und umweltschädliche Chemikalien zur Desinfektion eingesetzt werden müssen.

Ein weiterer Nachteil liegt in der Tatsache begründet, dass UV-Strahlung ein gesundheitliches Risiko für Menschen darstellen. UV-Strahlung kann in Abhängigkeit der Intensität für Menschen und andere Organismen gefährlich sein. Beispielsweise kann UV-Strahlung mit größerer Wellenlänge bereits chemische Bindungen organischer Moleküle zerstören. Daher ist aus gesundheitlichen Gründen Vorsicht sowohl im Umgang mit Sonnenlicht als auch mit technischen UV-Quellen angebracht.

### Darstellung der Erfindung

Aufgabe der Erfindung ist es, eine dem eingangs genannten technischen Gebiet zugehörende Vorrichtung zum Entkeimen von Fluid zu schaffen, welche eine verbesserte Effizienz und eine damit verbundene umweltfreundliche und risikoarme Entkeimung von Fluid, insbesondere Luft, ermöglicht.

Zudem ist es die Aufgabe der Erfindung eine Vorrichtung zum Entkeimen von Fluid bereitzustellen, die ohne den Einsatz von Chemikalien, Desinfektionsmittel und ohne die Abscheidung von Nebenprodukten wie Ozon funktioniert.

Die Lösung der Aufgabe ist durch die Merkmale des Anspruchs 1 definiert. Gemäss der Erfindung umfasst die Vorrichtung zum Entkeimen von Fluid ein Gehäuse, einen Fluideinlass zum Einströmen von zu entkeimendem Fluid in das Gehäuse, einen Fluidauslass zum Ausströmen von entkeimten Fluid aus dem Gehäuse, eine Brennkammer mit einer UV-Lampeneinheit zum Entkeimen von durchströmenden Fluid, wobei die Brennkammer innenseitig zumindest teilweise verspiegelt ausgebildet ist, und wobei zwischen der Brennkammer und einer ersten Gehäusewand ein Fluideinlasskanal sowie zwischen der Brennkammer und einer zweiten Gehäusewand ein Fluidauslasskanal angeordnet ist.

Durch die zumindest teilweise verspiegelte Innenseite der Brennkammer, kann die emittierte UV-Strahlung reflektiert und zur weiteren Entkeimung durchströmenden Fluids in der Brennkammer eingesetzt werden. Dadurch wird die Effizient der Vorrichtung gesteigert. Ein zusätzlicher technischer Vorteil wird durch die Anordnung des Fluideinlasskanals zwischen Brennkammer und der ersten Gehäusewand erreicht, da die innerhalb der Brennkammer emittierte UV-Strahlung nicht aus dem Gehäuse entweichen kann. Dies bewirkt, dass die Vorrichtung für Menschen gesundheitlich unbedenklich ist. Der identische Vorteil wird durch die entsprechende Anordnung des Fluidauslasskanals zwischen Brennkammer und der zweiten Gehäusewand erreicht.

Beispielsweise können sowohl der Fluideinlass und/ oder der Fluidauslass mit zusätzlichen lärmdämmenden Mitteln ausgekleidet werden, wodurch insgesamt eine Verringerung der Geräuschkulisse der erfindungsgemässen Vorrichtung erreicht werden kann.

Ultraviolettstrahlung, kurz UV, UV-Strahlung oder auch Schwarzlicht genannt, ist eine elektromagnetische Strahlung im optischen Frequenzbereich (Licht), wobei sie eine kürzere Wellenlängen als für den Menschen sichtbares Licht aufweist. Grundsätzlich reicht der UV-Bereich etwa von 380 nm bis 100 nm und wird in die Unterbereiche UV-A, UV-B und UV-C eingeteilt.

Diese Eigenschaft von UV-Strahlen eignet sich insbesondere zur Entkeimung von Luft und Wasser aber auch von Gegenständen und Lebensmitteln. Die erfindungsgemässe Vorrichtung ist vorzugsweise für den Einsatz von Raumluft geeignet. Jedoch ist sie keineswegs darauf beschränkt, da jede Form eines Fluids mit der Wirkungsweise von UV-Strahlung entkeimt werden kann.

Das Gehäuse der erfindungsgemässen Vorrichtung lässt sich sowohl zur Festinstallation oder auch als flexibles oder mobiles Standgerät konzipieren, wobei diese Konzeption keinen Einfluss auf die Wirkungsweise der Vorrichtung hat.

Gemäss einer bevorzugten Ausführungsform, ist die Brennkammer innenseitig vollständig verspiegelt ausgebildet. Durch das vollständige Verspiegeln der Innenseite der Brennkammer kann die Effizienz der Vorrichtung zusätzlich erhöht werden. Die Reflektion der emittierten UV-Strahlung wird reflektiert und zur weiteren Entkeimung durchströmenden Fluids in der Brennkammer eingesetzt. Beispielsweise eignet sich hierfür besonders ein eloxiertes Reflektorblech aus Aluminium, insbesondere ein hochglanz eloxiertes Reflektorblech aus Aluminium. Die Stärke des Aluminiumblechs beträgt im Besonderen 0,1 - 0,9 mm, beispielsweise 0,5 mm.

Nach einer weiteren Ausführungsform, weist die UV-Lampeneinheit zumindest eine UVC-Röhre auf, die entlang einer Fluiddurchströmungsrichtung angeordnet ist. Dadurch wird insbesondere der technische Vorteil erreicht, dass die Wirkungszeit der UVC-Strahlung auf das Fluid vergrössert wird. Aufgrund der Ausrichtung der Röhre in Fluidströmungsrichtung, wird Fluid, welches entlang der Röhre durch die Brennkammer strömt, länger und damit intensiver der UVC-Strahlung ausgesetzt.

UVC-Strahlung ist ein Bereich der UV-Strahlung und liegt hierbei im Intervall zwischen 280 nm und 100nm. UVC-Strahlung verändert die DNA/RNA von Mikroorganismen so, dass eine Reproduktion (Zellteilung) von Viren, Bakterien, Hefen sowie auch Schimmel nicht mehr möglich ist - sie werden inaktiviert und unschädlich gemacht. Dadurch ist UVC-Strahlung besonders für den eingesetzten Zweck geeignet. Die Reinigungswirkung und somit die Effizienz der Vorrichtung wird zusätzlich erhöht.

Gemäss einer zusätzlichen Ausführungsform, beträgt die Strahlungswellenlänge der UV-Lampeneinheit zwischen 240nm und 270nm. Nach einer besonderen Ausführungsform beträgt die Strahlungswellenlänge der UV-Lampeneinheit zwischen 250nm und 260nm. Nach einer weiteren besonderen Ausführungsform beträgt die Strahlungswellenlänge der UV-Lampeneinheit insbesondere zwischen 253nm und 254nm. Somit kann die UV-Lampeneinheit auch als UVC-Lampeneinheit bezeichnet werden. In den angegebenen Bereichen der Strahlungswellenlänge ist die Reinigungswirkung besonders hoch und der Wirkungsgrad beträgt bis zu 99,999 %. Beispielsweise ist eine 4 Pin 36 Watt Röhreneinheit mit einem Hi-End Vorschaltgerät für diesen Zweck besonders geeignet.

Nach einer besonderen Ausführungsform, weist das Gehäuse eine Fluidumwälzeinheit auf, welche zu entkeimendes Fluid durch den Fluideinlass und über den Fluideinlasskanal in die Brennkammer einbringt und entkeimtes Fluid über den Fluidauslasskanal und den Fluidauslass aus der Brennkammer ausbringt. Beispielsweise eignet sich ein schallgedämpfter Ventilator in halbradialer Bauweise als Fluidumwälzeinheit. In einer zentralen Ausführung der Umwälzeinheit lässt sich diese besonders einfach reparieren oder austauschen. Alternativ kann die Umwälzeinheit jedoch auch mehrere Umwälzmodule oder Gebläseeinheiten umfassen, welche in Abhängigkeit des Betriebsmodus vereinzelt oder parallel betrieben werden können. Diese Betriebsmodi hängen insbesondere von dem Zielkeim und von dem erwünschten Entkeimungsresultat ab und werden über eine zentrale Steuereinheit betrieben.

Um die Vorrichtung vorzugsweise in kleinen bis mittelgrossen Räumen einsetzen zu können, in welchen sich mehrere Personen aufhalten und begegnen, ist die Fluidumwälzeinheit dazu ausgebildet mehr als 1000 m³ /h, insbesondere mehr als 1500 m³ /h Fluid umzuwälzen. Darüber hinaus sind Umwälzleistungen von bis zu 1800 m³ /h möglich, wodurch eine effektive Desinfektion von Raumluft auch in mittelgrossen Räumen mit mehreren Personen in kurzer Zeit realisierbar ist.

Gemäss einer besonders bevorzugten Ausführungsform, ist in einer Seitenansicht der Vorrichtung der Verlauf des Fluidstroms durch das Gehäuse beginnend vom Fluideinlass über den Fluideinlasskanal in die Brennkammer über den Fluidauslasskanal und den Fluidauslass mäanderförmig ausgebildet. Die mäanderförmige Ausbildung des Verlaufs des Fluidstroms hat einerseits den technischen Vorteil, dass keine UV Strahlung aus der Brennkammer heraus an die Aussenseite des Gehäuses gelangen kann, wodurch insbesondere gesundheitsspezifischen Aspekte für Menschen im Umfeld der Vorrichtung Rechnung getragen wird. Dies gelingt insbesondere durch die antiparallele Anordnung des Fluideinlasskanals, der Brennkammer und des Fluidauslasskanals. Fluideinlasskanal, Brennkammer und Fluidauslasskanal sind parallel zueinander angeordnet, jedoch ist die Fluiddurchströmungsrichtung jeweils entgegengesetzt.

Zusätzlich kann aufgrund des mäanderförmigen Verlaufs auf zusätzliche Filtereinheiten verzichtet werden, wodurch sich der Widerstand der Vorrichtung und damit auch die Geräuschkulisse der Vorrichtung signifikant reduziert.

In eine weiteren Ausführungsform, weist die Brennkammer einen Brennkammereinlass zum Einströmen von zu entkeimenden Fluid aus dem Fluideinlasskanal und einen Brennkammerauslass zum Ausströmen von entkeimten Fluid in den Fluidauslasskanal auf.

Gemäss einer besonders bevorzugten Ausführungsform, ist der Fluideinlass gegenüber dem Brennkammereinlass versetzt angeordnet. Dadurch wird der technische Vorteil erreicht, dass ein Austritt von UV-Strahlung aus der Brennkammer heraus an die Aussenseite des Gehäuses vermieden werden kann. Hierbei können einerseits gesundheitsspezifische Aspekte für Personen im Umfeld der Vorrichtung Rechnung getragen werden. Andererseits lässt sich durch das Versetzen des Fluideinlasses gegenüber dem Brennkammereinlass der Einsatz von zusätzlichen Filtereinheiten vermeiden, was den Widerstand der Vorrichtung und damit auch die Geräuschkulisse der Vorrichtung reduziert.

Nach einer weiteren besonders bevorzugten Ausführungsform, ist der Fluidauslass gegenüber dem Brennkammerauslass versetzt angeordnet. Korrespondierend zur vorhergehenden Ausführungsform, wird dadurch ebenfalls der technische Vorteil erreicht, dass ein Austritt von UV-Strahlung aus der Brennkammer heraus an die Aussenseite des Gehäuses vermieden werden kann. Durch das Versetzen des Fluidauslasses gegenüber dem Brennkammerauslass kann der Einsatz von zusätzlichen Filtereinheiten vermieden werden, was den Widerstand der Vorrichtung und damit auch die Geräuschkulisse der Vorrichtung reduziert.

In einer weiteren Ausführungsform, ist der Fluideinlass in einem oberen Bereich, insbesondere in einem oberen Drittel, insbesondere in einem oberen Viertel des Gehäuses angeordnet ist. Dadurch kann beispielsweise der technische Vorteil erreicht werden, dass Aerosole einfacher direkt am Ort Ihres Entstehens in die Vorrichtung eingesaugt werden können. Häufig werden Aerosole von Menschen beim Ausatmen, beim Singen oder beim Husten erzeugt. Folglich entstehen derartige Aerosole in einer Höhe zwischen 1,5 m und 2 m. Beispielsweise kann das Gehäuse der erfindungsgemässen Vorrichtung in Grösse eines Schrankes ausgebildet sein. Wenn nun der Fluideinlass in einem oberen Bereich des Gehäuses - hier in Schrankgrösse - angeordnet ist, dann befindet sich der Fluideinlass in etwa auf Höhe des Ortes des Entstehens von menschlichen Aerosolen. Folglich kann ein besonders grosser Anteil eines Aerosols unmittelbar in die Vorrichtung eingesaugt werden, bevor es sich im Raum unkontrolliert verteilt. In der Konsequenz wird die Effizienz der Vorrichtung zusätzlich verbessert.

Gemäß einer vorteilhaften Ausführungsform, ist der Fluidauslass in einem unteren Bereich, insbesondere in einem unteren Drittel, insbesondere in einem unteren Viertel des Gehäuses angeordnet. Dadurch wird beispielsweise der technische Vorteil erreicht, dass das gereinigte Fluid keiner Person ins Gesicht geströmt wird. Stattdessen wird der Fluidstrom unbemerkt in Bodennähe aus dem Gehäuse ausgebracht.

Um die Reinigungsleistung der Vorrichtung zusätzlich zu verbessern, weist die Vorrichtung eine Kaltvernebelungseinheit zum Einbringen keimabtötender Zusätze in das Fluid auf.

Nach einer besonderen Ausführungsform, ist die Kaltvernebelungseinheit in dem Fluidauslasskanal angeordnet. Dies ermöglicht eine besonders kompakte Bauart der Vorrichtung, da keine gesonderten Kammern, Kanäle oder sonstige Gehäuseeinheiten hergestellt werden müssen. Beispielsweise wird der Fluidauslasskanal geringfügig erweitert, wodurch die Kaltvernebelungseinheit unmittelbar in dem Fluidauslasskanal angeordnet werden kann.

Beispielsweise in Laboren oder in ärztlichen Praxisräumen besteht das Bedürfnis erzeugte Aerosole in Abhängigkeit der gegebenen Situation oder eines Patienten aufzufangen. Mit anderen Worten ist es häufig eine grosse Herausforderung eine örtliche Flexibilität zur Aufnahme von Aerosolen sicherzustellen. Nach einer zusätzlichen Ausführungsform, weist die Vorrichtung ein Adapterelement auf, um eine Position eines weiteren Fluideinlasses relativ zum Gehäuse zu verändern. Dadurch wird beispielsweise der technische Vorteil erreicht, dass der weitere Fluideinlass manuell oder automatisch an die situativen Gegebenheiten angepasst werden kann. Beispielsweise kann der Fluideinlass am Ende einer Verrohrung oder eines Schlauches angeordnet sein. Hier kann ein Bediener - beispielsweise ein Zahnarzt - der Vorrichtung den weiteren Fluideinlass manuell auf einen liegenden Patienten - insbesondere dessen Mundöffnung - ausrichten. In ähnlicher Form ist das Prinzip auch auf Labore usw. übertragbar.

Gemäss einem weiteren Aspekt betrifft die Erfindung eine Verwendung einer Vorrichtung zum Entkeimen eines Fluids nach einer der vorausgehenden Ausführungsformen. Insbesondere betrifft dies die Verwendung einer Vorrichtung zum Entkeimen von Fluid, mit einem Gehäuse, einem Fluideinlass zum Einströmen von zu entkeimendem Fluid in das Gehäuse, einem Fluidauslass zum Ausströmen von entkeimten Fluid aus dem Gehäuse, einer Brennkammer mit einer UVC-Lampeneinheit zum Entkeimen von durchströmenden Fluid, wobei die Brennkammer innenseitig zumindest teilweise verspiegelt ausgebildet ist, und wobei zwischen der Brennkammer und einer ersten Gehäusewand ein Fluideinlasskanal sowie zwischen der Brennkammer und einer zweiten Gehäusewand ein Fluidauslasskanal angeordnet ist.

Es ergeben sich bei der Verwendung der Vorrichtung vergleichbare Vorteile wie bei der Vorrichtung selbst. Durch die zumindest teilweise verspiegelte Innenseite der Brennkammer, kann die emittierte UV-Strahlung reflektieren und zur weiteren Entkeimung durchströmenden Fluids in der Brennkammer eingesetzt werden. Dadurch wird die Effizienz der Vorrichtung gesteigert. Ein weiterer technischer Vorteil wird durch die Anordnung des Fluideinlasskanals zwischen Brennkammer und der ersten Gehäusewand erreicht, da die innerhalb der Brennkammer emittierte UV-Strahlung nicht aus dem Gehäuse entweichen kann. Dies bewirkt, dass die Vorrichtung für Personen gesundheitlich absolut unbedenklich ist. Der identische Vorteil wird durch die entsprechende Anordnung des Fluidauslasskanals zwischen Brennkammer und der zweiten Gehäusewand erreicht.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine Schnittansicht einer Vorrichtung zum Entkeimen von Fluid nach einer ersten Ausführungsform,
- Fig. 2: die Vorrichtung zum Entkeimen von Fluid in einer Explosionsdarstellung nach einer weiteren Ausführungsform,
- Fig. 3: die erfindungsgemässe Vorrichtung gemäss Figur 2 in einer alternativen Ansicht, und
- Fig. 4: eine Vorrichtung zum Entkeimen von Fluid in einer perspektivischen Ansicht nach einer weiteren Ausführungsform.

Grundsätzlich sind in den Figuren gleiche Teile mit gleichen Bezugszeichen versehen.

### Wege zur Ausführung der Erfindung

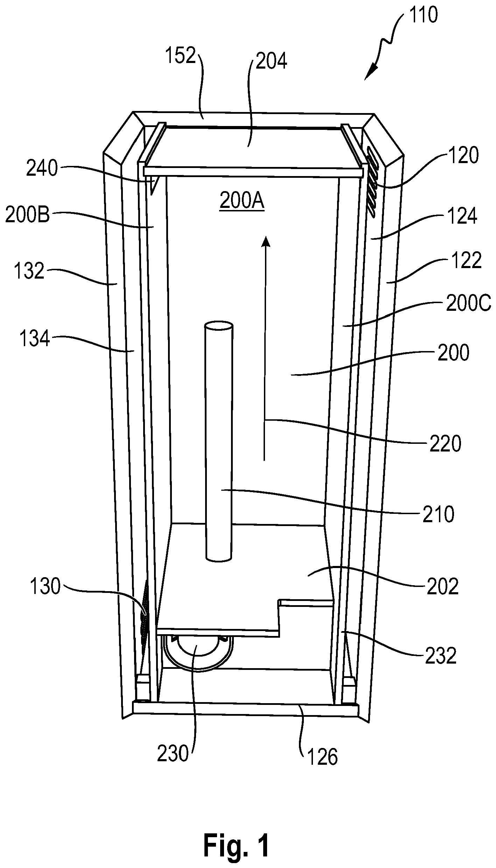

Die Figur 1 zeigt eine Schnittansicht einer Vorrichtung zum Entkeimen von Fluid in einer ersten Ausführungsform. Die Vorrichtung umfasst ein Gehäuse 110 mit einer ersten Gehäusewand 122 und einer zweiten Gehäusewand 132. Die erste Gehäusewand 122 und die zweite Gehäusewand 132 sind einander gegenüberliegend angeordnet. Die erste Gehäusewand 122 umfasst an ihrem oberen Ende einen Fluideinlass 120 zum Einströmen von zu entkeimendem Fluid in das Gehäuse 110. Die der ersten Gehäusewand 122 gegenüberliegende zweite Gehäusewand 132 umfasst an ihrem unteren Ende einen Fluidauslass 130 zum Ausströmen von entkeimten Fluid aus dem Gehäuse 110.

Innerhalb des Gehäuses 110 befindet sich eine Brennkammer 200 mit einer UV-Lampeneinheit 210 zum Entkeimen von durchströmenden Fluid. Die UV-Lampeneinheit 210 umfasst eine UVC-Röhre. Denkbar ist jedoch mehrere UVC-Röhren in der Brennkammer 200 anzuordnen, die idealerweise entlang der Fluiddurchströmungsrichtung 220 von zu entkeimenden Fluid angeordnet sind. Die Ausrichtung der UVC-Röhren ist hierbei derart orientiert, dass die Strahlung der UVC-Röhren eine möglichst grosse Einwirkungszeit auf das durchströmende Fluid aufweist. Beispielsweise können 2, 3 oder 4 UVC-Röhren in der Brennkammer angeordnet sein.

Die Innenwände 200A, 200B, 200C, 200D (teilweise nicht gezeigt) der Brennkammer 200 sind vollständig verspiegelt ausgebildet, um die Effizienz der Entkeimung durch eine Reflektion der emittierten UV-Strahlung zusätzlich zu steigern. An einem oberen Ende der Innenwand 200B der Brennkammer 200 befindet sich ein Brennkammerauslass 240 für entkeimtes Fluid, welches die Brennkammer 200 in Fluiddurchströmungsrichtung 220, also von unter nach oben durchströmt hat. An einem unteren Ende der Innenwand 200C der Brennkammer 200 befindet sich ein Brennkammereinlass 232 (nicht gezeigt) für zu entkeimendes Fluid.

Zwischen der Innenwand 200C der Brennkammer 200 und der ersten Gehäusewand 122 befindet sich ein Fluideinlasskanal 124. Der Fluideinlasskanal 124 verbindet den Fluideinlass 120 mit dem Brennkammereinlass 232. Zu entkeimendes Fluid tritt somit durch den Fluideinlass 120 in das Gehäuse 110 ein und strömt durch den Fluideinlasskanal 124 antiparallel zur Fluiddurchströmungsrichtung 220 entlang der Innenwand 200C durch den Brennkammereinlass 232 in die Brennkammer 200 ein.

Zwischen der Innenwand 200B der Brennkammer 200 und der zweiten Gehäusewand 132 befindet sich ein Fluidauslasskanal 134. Der Fluidauslasskanal 134 verbindet den Fluidauslass 130 mit dem Brennkammerauslass 240. Entkeimtes Fluid tritt somit durch den Brennkammerauslass 240 aus der Brennkammer 200 aus und strömt durch den Fluidauslasskanal 134 antiparallel zur Fluiddurchströmungsrichtung 220 entlang der Innenwand 200Bzum Fluidauslass 130 aus dem Gehäuse 110 heraus.

Unterhalb eines Brennkammerbodens 202 befindet sich eine Fluidumwälzeinheit 230. Die Fluidumwälzeinheit 230 saugt zu entkeimendes Fluid durch den Fluideinlass 120 über den Fluideinlasskanal 124 in die Brennkammer 200 ein, strömt das zu entkeimende Fluid in Fluiddurchströmungsrichtung 220 durch die Brennkammer 200 hindurch und bringt entkeimtes Fluid über den Brennkammerauslass 240 in den Fluidauslasskanal 134 und den Fluidauslass 130 aus dem Gehäuse 110 der Vorrichtung 100 aus. Bei Volllast ist die Fluidumwälzeinheit 230 dazu ausgebildet bis zu 1800 m³ /h Fluid durch die Vorrichtung 100 hindurch zu befördern, wodurch eine effektive Desinfektion von Raumluft auch in mittelgrossen Räumen mit mehreren Personen realisierbar ist.

Der Fluideinlass 120 in den ersten Gehäusewand 122 ist gegenüber dem Brennkammereinlass 232 versetzt angeordnet und der Fluidauslass 130 in der zweiten Gehäusewand 132 ist gegenüber dem Brennkammerauslass 240 versetzt angeordnet. In der Seitenansicht ergibt sich dadurch, dass der Strömungsweg des Fluides durch das Gehäuse 110 beginnend beim Einströmen in den Fluideinlass 120 über den Fluideinlasskanal 124 in die Brennkammer 200 und durch die Brennkammer 200 hindurch, über den Fluidauslasskanal 134 und den Fluidauslass 130 mäanderförmig verläuft. Hierbei entsteht der wichtige Vorteil, dass die Brennkammer 200 nach aussen vollständig abgeschirmt ist und UV-Strahlung nicht entweichen kann.

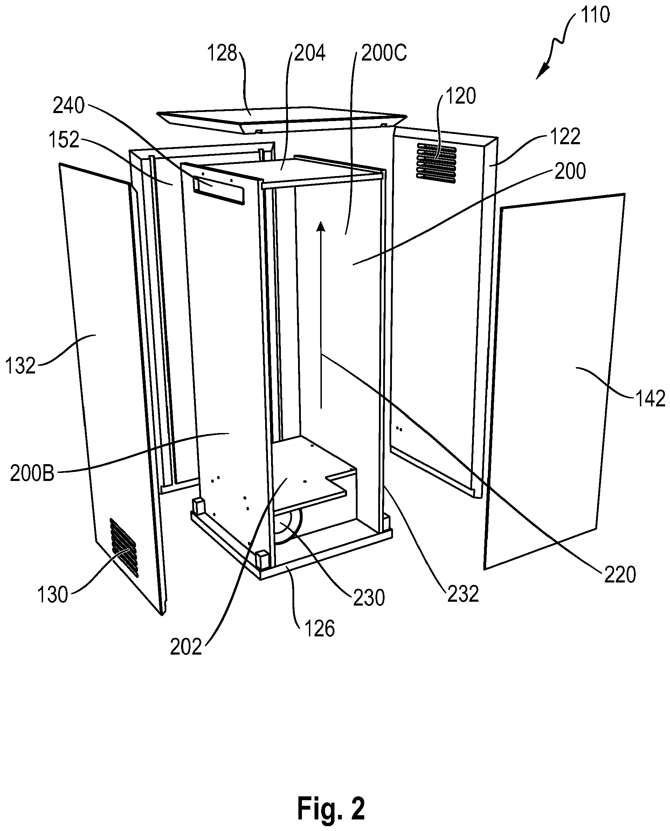

Die Figur 2 zeigt die Vorrichtung zum Entkeimen von Fluid in einer Explosionsdarstellung nach einer weiteren Ausführungsform.

Die Vorrichtung umfasst das Gehäuse 110 mit der ersten Gehäusewand 122, der zweiten Gehäusewand 132, einer dritten Gehäusewand 142 und einer vierten Gehäusewand 152. Die Oberseite des Gehäuses 110 weist einen Gehäusedeckel 126, während die Unterseite mit einem Gehäuseboden 126 abschliesst. Beispielsweise kann der Gehäuseboden 128 ein Fahrwerk aufweisen, wodurch die gesamte Vorrichtung auf dem Boden bewegbar ist. Mittels einer Blockierfunktion kann das Fahrwerk nach Erreichen der gewünschten Position fixiert werden.

Um die Vorrichtung zum Entkeimen von Fluid möglichst einfach zu reparieren oder zu reinigen, lässt sich die Brennkammer 200 aus dem Gehäuse 110 entfernen. Hierfür ist die Brennkammer 200 als austauschbares Modul ausgebildet. Das Entfernen der Brennkammer 200 aus dem Gehäuse 110 erfolgt translatorisch, nachdem zunächst die dritte Gehäusewand 142 von dem Gehäuse 110 getrennt wurde. Beispielsweise kann die Brennkammer 200 in dem Gehäuse 110 selbst mittels Schienen gelagert sein, um die translatorische Bewegung aus dem Gehäuse 110 heraus oder in das Gehäuse 110 hinein zu erleichtern.

Beispielsweise sind diejenigen Innenwände 200A, 200D der Brennkammer 110, welche der dritten Gehäusewand 142 und der vierten Gehäusewand 152 zugeordnet ist, unmittelbar an diesen Gehäusewänden fixiert.

Alle Bezugszeichen der Figur 2 bezeichnen identische Merkmale der vorausgehenden Figur. Auf eine wiederhole Beschreibung der identischen Merkmale wird verzichtet.

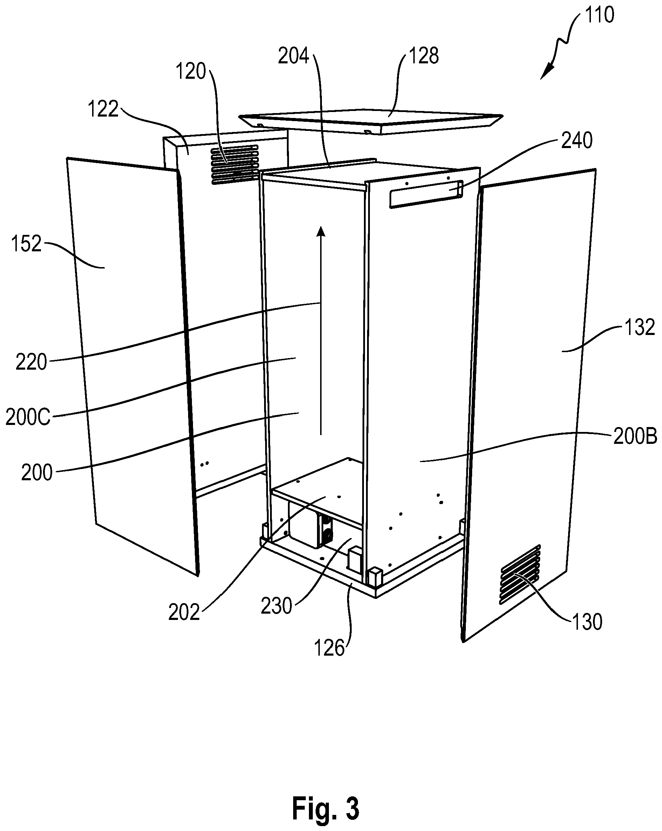

Die Figur 3 zeigt die erfindungsgemässe Vorrichtung gemäss Figur 1 und Figur 2 in einer alternativen Ansicht. Alle Bezugszeichen der Figur 3 bezeichnen identische Merkmale der vorausgehenden Figuren. Auf eine wiederhole Beschreibung der identischen Merkmale wird verzichtet.

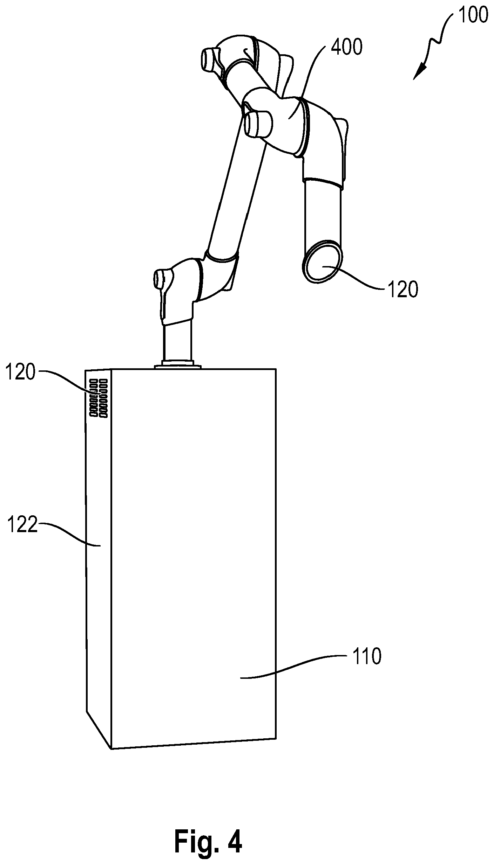

Die Figur 4 zeigt eine Vorrichtung 100 zum Entkeimen von Fluid in einer perspektivischen Ansicht nach einer weiteren Ausführungsform. Die Vorrichtung 100 umfasst das Gehäuse 110 mit der ersten, zweiten, dritten und vierten Gehäusewand 122, 132, 142, 152 (teilweise nicht gezeigt). Die erste Gehäusewand 122 umfasst an ihrem oberen Ende den Fluideinlass 120 zum Einströmen von zu entkeimenden Fluid in das Gehäuse 110. Die der ersten Gehäusewand 122 gegenüberliegende zweite Gehäusewand 132 (nicht gezeigt) umfasst an ihrem unteren Ende den Fluidauslass 130 (nicht gezeigt) zum Ausströmen von entkeimten Fluid aus dem Gehäuse 110.

Innerhalb des Gehäuses 110 befindet sich die Brennkammer mit einer UV-Lampeneinheit zum Entkeimen von durchströmenden Fluid. Die Innenwände der Brennkammer sind vollständig verspiegelt ausgebildet um die Effizienz der Entkeimung durch eine Reflektion der emittierten UV-Strahlung zusätzlich zu steigern.

Der Fluideinlass 120 befindet sich in einem oberen Bereich des Gehäuses 110, wodurch Aerosole einfacher am Ort Ihres gewöhnlichen Entstehens in die Vorrichtung 100 eingesaugt werden können. Zusätzlich weist die Vorrichtung 100 ein Adapterelement 400 mit mehreren gelenkig miteinander verbundenen Rohrelementen auf. An einem freien Ende des Adapterelements 400 befindet sich ein weiterer Fluideinlass 120 zum Ansaugen von zu entkeimenden Fluid. Der Vorteil des Adapterelements 400 liegt in der Veränderbarkeit der Position des weiteren Fluideinlasses 120 relativ zum Gehäuse 110. Im Inneren des Gehäuses 110 wird sowohl Fluid, welches über den Fluideinlass 120 in der ersten Gehäusewand 122 als auch Fluid welches über den Fluideinlass 120 am offenen Ende des Adapterelements 400 in das Gehäuse 110 der Vorrichtung 100 eingeströmt wird, durch die Brennkammer mit der UV-Lampeneinheit geströmt und entkeimt.

Der Einsatz des Adapterelements 400 eignet sich besonders in Laboren oder in ärztlichen Praxisräumen, wo Aerosole in Abhängigkeit der Situation oder von Patienten aufgefangen werden müssen.

Ein zusätzlicher Aspekt der Erfindung liegt in der Verwendung der Vorrichtung 100 zum Entkeimen eines Fluids. Die Verwendung umfasst sowohl die Vorrichtung 100 zum Entkeimen eines Fluids gemäss den Figuren 1 bis 3 als auch der Vorrichtung 100 zum Entkeimen eines Fluids mit Adapterelement 400 gemäss der Figur 4 und der Vorrichtung 100 zum Entkeimen eines Fluids einschliesslich Kaltvernebelungseinheit zum Einbringen keimabtötender Zusätze in das Fluid.

## Patentansprüche

1. Vorrichtung (100) zum Entkeimen von Fluid, insbesondere Luft, mit:
einem Gehäuse (110),
einem Fluideinlass (120) zum Einströmen von zu entkeimendem Fluid in das Gehäuse (110),
einem Fluidauslass (130) zum Ausströmen von entkeimten Fluid aus dem Gehäuse (110),
einer Brennkammer (200) mit einer UV-Lampeneinheit (210) zum Entkeimen von durchströmenden Fluid, wobei
die Brennkammer (200) innenseitig zumindest teilweise verspiegelt ausgebildet ist, und wobei
zwischen der Brennkammer (200) und einer ersten Gehäusewand (122) ein Fluideinlasskanal (124) sowie zwischen der Brennkammer (200) und einer zweiten Gehäusewand (132) ein Fluidauslasskanal (134) angeordnet ist.

2. Vorrichtung (100) nach Anspruch 1, wobei die Brennkammer (200) innenseitig vollständig verspiegelt ausgebildet ist.

3. Vorrichtung (100) nach Anspruch 1 oder 2, wobei die UV-Lampeneinheit (210) zumindest eine UVC-Röhre aufweist, die entlang einer Fluiddurchströmungsrichtung (220) angeordnet ist.

4. Vorrichtung (100) nach einem der Ansprüche 1 bis 3, wobei die Strahlungswellenlänge der UV-Lampeneinheit (210) zwischen 240nm und 270nm, insbesondere zwischen 250nm und 260nm, insbesondere zwischen 253nm und 254nm beträgt.

5. Vorrichtung (100) nach einem der voranstehenden Ansprüche, wobei das Gehäuse (110) eine Fluidumwälzeinheit (230) aufweist, welche zu entkeimendes Fluid durch den Fluideinlass (120) und über den Fluideinlasskanal (124) in die Brennkammer (200) einbringt und entkeimtes Fluid über den Fluidauslasskanal (134) und den Fluidauslass (130) aus der Brennkammer (200) ausbringt.

6. Vorrichtung (100) nach Anspruch 5, wobei die Fluidumwälzeinheit (230) dazu ausgebildet ist mehr als 1000 m³ /h, insbesondere mehr als 1500 m³ /h Fluid umzuwälzen.

7. Vorrichtung (100) nach einem der voranstehenden Ansprüche, wobei in einer Seitenansicht der Vorrichtung (100) der Verlauf des Fluidstroms durch das Gehäuse (110) beginnend vom Fluideinlass (120) über den Fluideinlasskanal (124) in die Brennkammer (200) über den Fluidauslasskanal (134) und den Fluidauslass (130) mäanderförmig ausgebildet ist.

8. Vorrichtung (100) nach einem der voranstehenden Ansprüche, wobei die Brennkammer (200) einen Brennkammereinlass (232) zum Einströmen von zu entkeimenden Fluid aus dem Fluideinlasskanal (124) und einen Brennkammerauslass (240) zum Ausströmen von entkeimten Fluid in den Fluidauslasskanal (134) aufweist.

9. Vorrichtung (100) nach Anspruch 8, wobei der Fluideinlass (120) gegenüber dem Brennkammereinlass (230) versetzt angeordnet ist.

10. Vorrichtung (100) nach Anspruch 8 oder 9, wobei der Fluidauslass (130) gegenüber dem Brennkammerauslass (240) versetzt angeordnet ist.

11. Vorrichtung (100) nach einem der voranstehenden Ansprüche, wobei der Fluideinlass (120) in einem oberen Bereich, insbesondere in einem oberen Drittel, insbesondere in einem oberen Viertel des Gehäuses (110) angeordnet ist.

12. Vorrichtung (100) nach einem der voranstehenden Ansprüche, wobei der Fluidauslass (130) in einem unteren Bereich, insbesondere in einem unteren Drittel, insbesondere in einem unteren Viertel des Gehäuses (110) angeordnet ist.

13. Vorrichtung (100) nach einem der voranstehenden Ansprüche, wobei die Vorrichtung (100) eine Kaltvernebelungseinheit (300) zum Einbringen keimabtötender Zusätze in das Fluid aufweist, wobei die Kaltvernebelungseinheit (300) bevorzugt in dem Fluidauslasskanal (134) angeordnet ist.

14. Vorrichtung (100) nach einem der voranstehenden Ansprüche, wobei die Vorrichtung (100) ein Adapterelement (400) aufweist, um eine Position eines weiteren Fluideinlasses (120) relativ zum Gehäuse (110) zu verändern.

15. Verwendung einer Vorrichtung (100) nach einem der Ansprüche 1 bis 14 zum Entkeimen eines Fluids.
